(19) Europäisches Patentamt European Patent Office Office européen des brevets

(11) **EP 2 099 475 B1**

(12) **EUROPEAN PATENT SPECIFICATION**

(45) Date of publication and mention of the grant of the patent:
**24.08.2016 Bulletin 2016/34**

(21) Application number: **08707824.2**

(22) Date of filing: **03.01.2008**

(51) Int Cl.:
*A61K 38/36* (2006.01)     *A61P 7/04* (2006.01)

(86) International application number:
**PCT/EP2008/050031**

(87) International publication number:
**WO 2008/081024 (10.07.2008 Gazette 2008/28)**

(54) **SUBCUTANEOUS ADMINISTRATION OF COAGULATION FACTOR VIIA-RELATED POLYPEPTIDES**

SUBKUTANE VERABREICHUNG VON POLYPEPTIDEN IN ZUSAMMENHANG MIT DEM GERINNUNGSFAKTOR VIIA

ADMINISTRATION SOUS-CUTANÉE DE POLYPEPTIDES LIÉS AU FACTEUR VIIA DE LA COAGULATION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.01.2007 EP 07000057**
**04.01.2007 US 878576 P**

(43) Date of publication of application:
**16.09.2009 Bulletin 2009/38**

(73) Proprietor: **Novo Nordisk Health Care AG**
**Thurgauerstrasse 36/38**
**8050 Zürich (CH)**

(72) Inventors:
• **RISCHEL, Christian**
**DK-2300 Copenhagen S (DK)**
• **SØRENSEN, BINOW, Brit**
**DK-3460 Birkeroed (DK)**
• **STENNICKE, Henning, Ralf**
**DK-2980 Kokkedal (DK)**

(74) Representative: **Winther, Kamilla**
**Novo Nordisk A/S**
**Corporate Patents**
**Novo Allé**
**2880 Bagsvaerd (DK)**

(56) References cited:
WO-A-01/82943          WO-A-2004/000366
WO-A1-02/077218       US-A1- 2002 137 673
US-A1- 2003 054 018    US-A1- 2003 104 978
US-A1- 2003 199 444    US-A1- 2007 254 840

• STONE MATTHEW ET AL: 'Unusual benefits of macromolecular shielding by polyethylene glycol for reactions at the diffusional limit: the case of factor VIIai and tissue factor.' BIOCHEMISTRY vol. 41, no. 52, 31 December 2002, pages 15820 - 15825, XP055034618 ISSN: 0006-2960
• DATABASE BIOSIS [Online] BIOSCIENCES INFORMATION SERVICE, PHILADELPHIA, PA, US November 2007 GHOSH SAMIT ET AL: 'Activity and regulation of long-acting factor VIIa analogs' Database accession no. PREV200800218413 & BLOOD, vol. 110, no. 11, Part 1, November 2007 (2007-11), page 924A, 49TH ANNUAL MEETING OF THE AMERICAN-SOCIETY-OF-HEMATOLOGY; ATLANTA, GA, USA; DECEMBER 08 -11, 2007 ISSN: 0006-4971

**Description**

**FIELD OF THE INVENTION**

The invention relates to the use of Factor VIIa-related polypeptides for the manufacture of a medicament for prevention or treatment of conditions affectable by Factor VIIa or the Factor VIIa-related polypeptide, wherein the medicament is for subcutaneous or intramuscular administration.

**BACKGROUND OF INVENTION**

Blood coagulation is a process consisting of a complex interaction of various blood components, or factors, which eventually gives rise to a fibrin clot. Generally, the blood components that participate in what has been referred to as the coagulation "cascade" are proenzymes or zymogens, enzymatically inactive proteins that are converted to proteolytic enzymes by the action of an activator, which is itself an activated clotting Factor. Coagulation factors that have undergone such a conversion are generally referred to as "active factors", and are designated by the addition of a lower case "a" suffix (e.g., Factor VIIa).

Activated Factor X ("Xa") is required to convert prothrombin to thrombin, which then converts fibrinogen to fibrin as a final stage in forming a fibrin clot. There are two systems, or pathways, that promote the activation of Factor X. The "intrinsic pathway" refers to those reactions that lead to thrombin formation through utilisation of factors present only in plasma. A series of protease-mediated activations ultimately generates Factor IXa, which, in conjunction with Factor VIIIa, cleaves Factor X into Xa. An identical proteolysis is effected by Factor VIIa and its co-Factor, tissue factor, in the "extrinsic pathway" of blood coagulation. Tissue factor is a membrane bound protein and does not normally circulate in plasma. Upon vessel disruption, however, it can complex with Factor VIIa to catalyse Factor X activation or Factor IX activation in the presence of $Ca^{++}$ and phospholipid. While the relative importance of the two coagulation pathways in haemostasis is unclear, in recent years Factor VII and tissue factor have been found to play a pivotal role in the regulation of blood coagulation.

Factor VII is a trace plasma glycoprotein that circulates in blood as a single-chain zymogen. The zymogen is catalytically inactive. Single-chain Factor VII may be converted to two-chain Factor VIIa by Factor Xa, Factor XIIa, Factor IXa or thrombin *in vitro.* Factor Xa is believed to be the major physiological activator of Factor VII. Like several other plasma proteins involved in haemostasis, Factor VII is dependent on vitamin K for its activity, which is required for the $\gamma$-carboxylation of multiple glutamic acid residues that are clustered in the amino terminus of the protein. These $\gamma$-carboxylated glutamic acids are required for the metal-associated interaction of Factor VII with phospholipids.

The conversion of zymogen Factor VII into the activated two-chain molecule occurs by cleavage of an internal peptide bond located approximately in the middle of the molecule. In human Factor VII, the activation cleavage site is at $Arg_{152}$-$Ile_{153}$. In the presence of tissue factor, phospholipids and calcium ions, the two-chain Factor VIIa rapidly activates Factor X or Factor IX by limited proteolysis.

Recombinant human Factor VIIa (rFVIIa) is an activated coagulation factor that is useful in the treatment of haemophilia patients who generate neutralising antibodies against Factor VIII or Factor IX. Administration of Factor VIII and Factor IX causes severe antibody formation in approximately 30% of the severe haemophilia patients. The action of rFVIIa (activation of the coagulation system via Factor X) is exerted in the vascular compartment of the body. The route of administration of rFVIIa has until now been intravenously. As a result of the relatively short half-life, administration normally has to be repeated every 2.5 to 3 hours. An alternative form of administration which would result in a reasonable bioavailability with a long lasting absorption phase would allow an increase in dosing intervals and at the same time make self administration possible, thus increasing the convenience for the patient.

Factor VIIa is a glycoprotein with a molecular weight of approximately 50 kDa. Factor VIIa has conventionally been delivered intravenously to haemophilia A or B patients, either prophylactically (such as, e.g., in anticipation of planned surgery) or in response to bleeding episodes. Such repeated use of intravenous injections, while necessary to control the disease, may have side effects. Repeated injections may lead to the vein at the site of injection becoming fibrosed or occluded a problem especially acute when treating the elderly. Also, when veins are small, as in babies/infants/toddlers, it may be difficult for the doctor/parents to insert a needle into the vein to inject the required therapeutic dose. A further impediment to intravenous administration is the prolonged time required for the infusion, which can be problematic when the patient is a child.

Coagulation Factors VIII (170 - 300 kDa) and IX (60 kDa) may be administered subcutaneously in the form of the single-chain zymogens, i.e., polypeptides not yet been activated. These non-activated forms are more stable than the activated (cleaved) forms, which are degraded much faster.

Factor VIIa is useful for administration to mammals, particularly humans, to control bleeding disorders, particularly bleeding disorders which are caused by clotting factor deficiencies (haemophilia A and B), or clotting factor inhibitors or bleeding disorders in patients not suffering from haemophilia A or B, for example, in patients

suffering from von Willebrand's disease. Patients with von Willebrand's disease have a defective primary haemostasis because they lack or have an abnormal von Willebrand factor protein. Bleeding disorders are also seen in patients with a normally functioning blood clotting cascade and may be caused by a defective platelet function, thrombocytopenia, or even by unknown reasons. Furthermore, FVIIa may be used for preventing or treating excessive bleedings in other patients, such as, for example, bleedings in association with tissue damage, for example surgery or trauma, especially in tissues rich in tissue factor (TF). FVIIa may be used as well as when the bleeding is diffuse and poorly responding to current haemostatic techniques and therapies (such as, e.g. haemorrhagic gastritis and profuse uterine bleeding). FVIIa may also be suitable for the treatment of bleedings occurring in organs with limited possibility for mechanical haemostasis such as brain, inner ear region, eyes as well as in association with the process of taking biopsies from various organs and in laparoscopic surgery.

International Patent Application No. WO 93/07890 relates to the treatment of haemophilia with FIX by subcutaneous injection.

International Patent Application No. WO 95/26750 relates to a formulation of FVIII or FIX suitable for subcutaneous injection for treatment of haemophilia A or B.

International Patent Application No. WO 95/28954 relates to concentrated preparations of FIX suitable for storage and subcutaneous injection.

International Patent Application No. WO 01/82943 relates to use of coagulation Factor VIIa for the manufacture of a medicament for prevention or treatment of conditions affectable by Factor VIIa.

International Patent Application No. WO 04/000366 relates to compositions comprising Factor VII conjugates having predetermined patterns of glycosylation.

Stone M. *et al.* discloses active site blocked wild-type factor VIIai and mutated factor VIIai modified with PEG-40KDa for anticoagulation therapy.

## BRIEF DESCRIPTION OF THE DRAWINGS

**Figure 1** illustrates the plasma concentration, ELISA (ng/ml) as a function of time (h) for the studied compounds after s.c. administration in minipigs (mean $\pm$ SD, n=2) (1; 2)

**Figure 2** illustrates the plasma FVIIa equivalent clot concentration (ng/ml) as a function of time (h) for the studied compounds after subcutaneous administration in minipigs (mean $\pm$ SD, n=2) (1; 2)

**Figure 3** Illustrates the plasma FVIIa concentration (ng/ml) as determined via ELISA as a function of time (h) for the studied compounds after s.c. administration in mice (mean $\pm$ SD)

**Figure 4** illustrates the plasma clot activity (ng/ml) as a function of time (h) for the studied compounds (mean $\pm$ SD) after s.c. administration to mice.

## SUMMARY OF THE INVENTION

The invention is directed to a PEGylated human Factor VIIa polypeptide for the use in prevention or treatment of a bleeding episode, wherein said polypeptide is for administering via a subcutaneous or intramuscular route, and wherein the average molecular weight of the polyethylene glycol moiety is 40 KDa.

Disclosed herein are methods for preventing or treating a bleeding episode which are carried out by administering to a patient in need of such treatment an effective amount for such treatment of a Factor VIIa-related polypeptide via subcutaneous or intramuscular route of administration.

Also disclosed is the use of a Factor VIIa-related polypeptide for the preparation of a medicament for preventing or treating a bleeding episode, wherein said preparation is suitable for administering via a subcutaneous or intramuscular route.

In some embodiments, the Factor VIIa-related polypeptide is an amino acid sequence variant of Factor VIIa. In some embodiments, the Factor VIIa-related polypeptide is a wild-type Factor VIIa or a sequence variant of Factor VIIa that has been modified to incorporate additional chemical moieties that are covalently or non-covalently bound to the polypeptide portion of the molecule. The binding may be directly to polypeptide portion, or, alternatively, indirectly, such as, e.g., via an oligosaccharide moiety. In some embodiments, the Factor VIIa-related polypeptide is modified by PEGylation, in which one or more polyethylene glycol (PEG) groups are conjugated either to the polypeptide chain directly (such as, e.g., by linkage to a cysteine residue) or via a protein-bound oligosaccharide. In some embodiments, the Factor VIIa-related polypeptides exhibit a bioavailability of at least about 125% of the bioavailability of wild-type (unmodified) Factor VIIa.

## DETAILED DESCRIPTION OF THE INVENTION

The subcutaneous or intramuscular 30 administration of Factor VIIa-related polypeptides, i.e., polypeptides that have been modified relative to wild-type Factor VIIa to enhance their therapeutic applications. It has now been found that such Factor VIIa-related polypeptides can be effectively administered via a subcutaneous route, providing therapeutically beneficial methods for preventing and/or treating Factor VIIa-responsive syndromes, such as, e.g., bleeding.

Factor VIIa-Related Polypeptides

Wild-type human Factor VII is a polypeptide having the amino acid sequence disclosed in U.S. Patent No. 4,784,950 (SEQ ID NO:1). The terms "Factor VIIa" and "Factor VIIa-related polypeptides" are intended to encompass polypeptides that have been proteolytically processed to yield their respective bioactive forms. Typically, Factor VII is cleaved between residues 152 and 153 to yield Factor VIIa.

The biological activity of Factor VIIa in blood clotting derives from its ability to (i) bind to tissue factor (TF) and (ii) catalyze the proteolytic cleavage of Factor IX or Factor X to produce activated Factor IX or X (Factor IXa or Xa, respectively). Factor VIIa biological activity may be quantified by measuring the ability of a preparation to promote blood clotting using Factor VII-deficient plasma and thromboplastin (or recombinant TF), as described, e.g., in U.S. Patent No. 5,997,864. In this assay, biological activity is expressed as the reduction in clotting time relative to a control sample and is converted to "Factor VII units" by comparison with a pooled human serum standard containing 1 unit/ml Factor VII activity. FVIIa activity may also be expressed in mg/ml when measured against a reference material with a known protein concentration and a known specific activity. Alternatively, Factor VIIa biological activity may be quantified by (i) measuring the ability of Factor VIIa to produce of Factor Xa in a system comprising TF embedded in a lipid membrane and Factor X. (Persson et al., J. Biol. Chem. 272:19919-19924, 1997); (ii) measuring Factor X hydrolysis in an aqueous system; (iii) measuring its physical binding to TF using an instrument based on surface plasmon resonance (Persson, FEBS Letts. 413:359-363, 1997) and (iv) measuring hydrolysis of a synthetic substrate. It will be understood that any method may be used to quantify Factor VIIa activity, when that method can be correlated with any of the above methods and assay units adjusted accordingly.

Factor VIIa-related polypeptides encompass polypeptides that differ from wild-type Factor VIIa in one or more of the following: (i) They contain variations in amino acid sequence (including the incorporation of modified amino acids); and/or (ii) they have been modified to incorporate additional chemical moieties that are covalently or non-covalently bound to the polypeptide portion. Such variants of Factor VII may exhibit different properties relative to human Factor VII, including stability, phospholipid binding, altered specific activity, and the like.

Non-limiting examples of Factor VII variants include S52A-FVIIa, S60A-FVIIa ( Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189 (corresponding to WO 02/077218); and FVII variants exhibiting increased proteolytic stability as disclosed in WO 02/38162 (Scripps Research Institute); FVII variants having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and FVII variants as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of FVII variants further include FVII variants as disclosed in WO 01/83725, WO 02/22776, WO 02/077218, PCT/DK02/00635 (corresponding to WO 03/027147), Danish patent application PA 2002 01423 (corresponding to WO 04/029090), Danish patent application PA 2001 01627 (corresponding to WO 03/027147); WO 02/38162 (Scripps Research Institute); and FVIIa variants with enhanced activity as disclosed in JP 2001061479 (Chemo-Sero-Therapeutic Res Inst.).

Non-limiting examples of Factor VIIa sequence variants further include: L305V, L305V/M306D/D309S, L305I, L305T, F374P, V158T/M298Q, V158D/E296V/M298Q, K337A, M298Q, V158D/M298Q, L305V/K337A, V158D/E296V/M298Q/L305V, V158D/E296V/M298Q/K337A, V158D/E296V/M298Q/L305V/K337A, K157A, E296V, E296V/M298Q, V158D/E296V, V158D/M298K, and S336G, L305V/K337A, L305V/V158D, L305V/E296V, L305V/M298Q, L305V/V158T, L305V/K337A/V158T, L305V/K337A/M298Q, L305V/K337A/E296V, L305V/K337A/V158D, L305V/V158D/M298Q, L305V/V158D/E296V, L305V/V158T/M298Q, L305V/V158T/E296V, L305V/E296V/M298Q, L305V/V158D/E296V/M298Q, L305V/V158T/E296V/M298Q, L305V/V158T/K337A/M298Q, L305V/V158T/E296V/K337A, L305V/V158D/K337A/M298Q, L305V/V158D/E296V/K337A, L305V/V158D/E296V/M298Q/K337A, L305V/V158T/E296V/M298Q/K337A, S314E/K316H, S314E/K316Q, S314E/L305V, S314E/K337A, S314E/V158D, S314E/E296V, S314E/M298Q, S314E/V158T, K316H/L305V, K316H/K337A, K316H/V158D, K316H/E296V, K316H/M298Q, K316H/V158T, K316Q/L305V, K316Q/K337A, K316Q/V158D, K316Q/E296V, K316Q/M298Q, K316Q/V158T, S314E/L305V/K337A, S314E/L305V/V158D, S314E/L305V/E296V, S314E/L305V/M298Q, S314E/L305V/V158T, S314E/L305V/K337A/V158T, S314E/L305V/K337A/M298Q, S314E/L305V/K337A/E296V, S314E/L305V/K337A/V158D, S314E/L305V/V158D/M298Q, S314E/L305V/V158D/E296V, S314E/L305V/V158T/M298Q, S314E/L305V/V158T/E296V, S314E/L305V/E296V/M298Q, S314E/L305V/V158D/E296V/M298Q, S314E/L305V/V158T/E296V/M298Q, S314E/L305V/V158T/K337A/M298Q, S314E/L305V/V158T/E296V/K337A, S314E/L305V/V158D/K337A/M298Q, S314E/L305V/V158D/E296V/K337A, S314E/L305V/V158D/E296V/M298Q/K337A, S314E/L305V/V158T/E296V/M298Q/K337A, K316H/L305V/K337A, K316H/L305V/V158D, K316H/L305V/E296V, K316H/L305V/M298Q, K316H/L305V/V158T, K316H/L305V/K337A/V158T, K316H/L305V/K337A/M298Q, K316H/L305V/K337A/E296V, K316H/L305V/K337A/V158D, K316H/L305V/V158D/M298Q, K316H/L305V/V158D/E296V, K316H/L305V/V158T/M298Q, K316H/L305V/V158T/E296V, K316H/L305V/E296V/M298Q, K316H/L305V/V158D/E296V/M298Q, K316H/L305V/V158T/E296V/M298Q, K316H/L305V/V158T/K337A/M298Q, K316H/L305V/V158T/E296V/K337A, K316H/L305V/V158D/K337A/M298Q, K316H/L305V/V158D/E296V/K337A, K316H/L305V/V158D/E296V/M298Q/K337A, K316H/L305V/V158T/E296V/M298Q/K337A, K316Q/L305V/K337A, K316Q/L305V/V158D, K316Q/L305V/E296V, K316Q/L305V/M298Q, K316Q/L305V/V158T, K316Q/L305V/K337A/V158T, K316Q/L305V/K337A/M298Q, K316Q/L305V/K337A/E296V, K316Q/L305V/K337A/V158D, K316Q/L305V/V158D/M298Q, K316Q/L305V/V158D/E296V, K316Q/L305V/V158T/M298Q, K316Q/L305V/V158T/E296V, K316Q/L305V/E296V/M298Q, K316Q/L305V/V158D/E296V/M298Q, K316Q/L305V/V158T/E296V/M298Q, K316Q/L305V/V158T/K337A/M298Q, K316Q/L305V/V158T/E296V/K337A, K316Q/L305V/V158D/K337A/M298Q, K316Q/L305V/V158D/E296V/K337A, K316Q/L305V/V158D/E296V/M298Q/K337A, K316Q/L305V/V158T/E296V/M298Q/K337A, F374Y/K337A, F374Y/V158D, F374Y/E296V, F374Y/M298Q, F374Y/V158T, F374Y/S314E, F374Y/L305V, F374Y/L305V/K337A, F374Y/L305V/V158D, F374Y/L305V/E296V, F374Y/L305V/M298Q, F374Y/L305V/V158T, F374Y/L305V/S314E, F374Y/K337A/S314E, F374Y/K337A/V158T, F374Y/K337A/M298Q, F374Y/K337A/E296V, F374Y/K337A/V158D, F374Y/V158D/S314E, F374Y/V158D/M298Q, F374Y/V158D/E296V, F374Y/V158T/S314E, F374Y/V158T/M298Q, F374Y/V158T/E296V, F374Y/E296V/S314E, F374Y/S314E/M298Q, F374Y/E296V/M298Q, F374Y/L305V/K337A/V158D, F374Y/L305V/K337A/E296V, F374Y/L305V/K337A/M298Q, F374Y/L305V/K337A/V158T, F374Y/L305V/K337A/S314E, F374Y/L305V/V158D/E296V, F374Y/L305V/V158D/M298Q, F374Y/L305V/V158D/S314E, F374Y/L305V/E296V/M298Q, F374Y/L305V/E296V/V158T, F374Y/L305V/E296V/S314E, F374Y/L305V/M298Q/V158T, F374Y/L305V/M298Q/S314E, F374Y/L305V/V158T/S314E, F374Y/K337A/S314E/V158T, F374Y/K337A/S314E/M298Q, F374Y/K337A/S314E/E296V, F374Y/K337A/S314E/V158D, F374Y/K337A/V158T/M298Q, F374Y/K337A/V158T/E296V, F374Y/K337A/M298Q/E296V, F374Y/K337A/M298Q/V158D, F374Y/K337A/E296V/V158D, F374Y/V158D/S314E/M298Q, F374Y/V158D/S314E/E296V, F374Y/V158D/M298Q/E296V, F374Y/V158T/S314E/E296V, F374Y/V158T/S314E/M298Q, F374Y/V158T/M298Q/E296V, F374Y/E296V/S314E/M298Q, F374Y/L305V/M298Q/K337A/S314E, F374Y/L305V/E296V/K337A/S314E, F374Y/E296V/M298Q/K337A/S314E, F374Y/L305V/E296V/M298Q/K337A, F374Y/L305V/E296V/M298Q/S314E, F374Y/V158D/E296V/M298Q/K337A, F374Y/V158D/E296V/M298Q/S314E, F374Y/L305V/V158D/K337A/S314E, F374Y/V158D/M298Q/K337A/S314E, F374Y/V158D/E296V/K337A/S314E, F374Y/L305V/V158D/E296V/M298Q, F374Y/L305V/V158D/M298Q/K337A, F374Y/L305V/V158D/E296V/K337A, F374Y/L305V/V158D/M298Q/S314E, F374Y/L305V/V158D/E296V/S314E, F374Y/V158T/E296V/M298Q/K337A, F374Y/V158T/E296V/M298Q/S314E, F374Y/L305V/V158T/K337A/S314E, F374Y/V158T/M298Q/K337A/S314E, F374Y/V158T/E296V/K337A/S314E, F374Y/L305V/V158T/E296V/M298Q, F374Y/L305V/V158T/M298Q/K337A, F374Y/L305V/V158T/E296V/K337A, F374Y/L305V/V158T/M298Q/S314E, F374Y/L305V/V158T/E296V/S314E, F374Y/E296V/M298Q/K337A/V158T/S314E, F374Y/V158D/E296V/M298Q/K337A/S314E, F374Y/L305V/V158D/E296V/M298Q/S314E, F374Y/L305V/E296V/M298Q/V158T/S314E, F374Y/L305V/E296V/M298Q/K337A/V158T, F374Y/L305V/E296V/K337A/V158T/S314E, F374Y/L305V/M298Q/K337A/V158T/S314E, F374Y/L305V/V158D/E296V/M298Q/K337A, F374Y/L305V/V158D/E296V/K337A/S314E, F374Y/L305V/V158D/M298Q/K337A/S314E, F374Y/L305V/E296V/M298Q/K337A/V158T/S314E, F374Y/L305V/V158D/E296V/M298Q/K337A/S314E, S52A-Factor VII, S60A-Factor VII; R152E-Factor VII, S344A-Factor VII, and P11Q/K33E, T106N, K143N/N145T, V253N, R290N/A292T, G291N, R315N/V317T, K143N/N145T/R315N/V317T; and FVII having substitutions, additions or deletions in the amino acid sequence from 233Thr to 240Asn, FVII having substitutions, additions or deletions in the amino acid sequence from 304Arg to 329Cys.

Modification of wild-type Factor VIIa or Factor VIIa sequence variants includes, without limitation, chemical and/or enzymatic modification, such as, e.g. by one or more of alkylation, glycosylation, PEGylation, acylation, phosphorylation, sulfation, or other ester formation or amide formation or the like. Examples include but are not limited to PEGylated wild-type human Factor VIIa, cysteine-PEGylated human Factor VIIa and variants thereof. Non-limiting examples of Factor VII derivatives include glycoPegylated FVII derivatives as disclosed in WO 2005/014035 (Novo Nordisk A/S); WO 03/31464 and US Patent applications US 20040043446, US 20040063911, US 20040142856, US 20040137557, and US 20040132640 (Neose Technologies, Inc.); other FVII conjugates are disclosed in WO 01/04287, US patent application 20030165996, WO 01/58935, WO 03/93465 (Maxygen ApS) and WO 02/02764, US patent application 20030211094 (University of Minnesota).

PEGylated human Factor VIIa encompasses any Factor VIIa to which one or more polyethylene glycol (PEG) moieties has been attached. The PEG molecule may be attached to any part of the Factor VIIa polypeptide, including any amino acid residue or carbohydrate moiety of the Factor VIIa polypeptide. The term "cysteine-PEGylated human Factor VIIa" refers to Factor VIIa having a PEG molecule conjugated to a sulfhydryl group of a cysteine introduced in human Factor VIIa to form a Factor VIIa sequence variant.

The Factor VIIa-related polypeptides have been modified to incorporate additional chemical moieties that are covalently or non-covalently bound to the polypeptide portion. In one embodiment such additional chemical moiety is selected from the list consisting of dendrimer, polyalkylene oxide (PAO), including polyalkylene glycol (PAG), such as polyethylene glycol (PEG) and polypropylene glycol (PPG), branched PEG, polyvinyl alcohol (PVA), polycarboxylate, poly-vinylpyrolidone, polyethylene-co-maleic acid anhydride, polystyrene-co-maleic acid anhydride, dextran, carboxymethyl-dextran.

The Factor VIIa-retated polypeptides have been modified to incorporate a polyethylene glycol (PEG), which may optionally be branched. In one embodiment the polyethylene glycol has an avarage molecular weight of 2-100 KDa, such as from 4-40 KDa, such as 5 KDa, 10 KDa, 20 KDa, or 40 KDa.

Factor VIIa-related polypeptides encompass those that exhibit at least about 5%, such as at least about 25%, such as at least about 50%, 75% and 90% of the specific activity of Factor VIIa that has been produced in the same cell type, when tested in one or more of a clotting assay, proteolysis assay, or TF binding assay as described above. Factor VII variants having a substantially modified biological activity relative to wild-type Factor VII include, without limitation, Factor VII variants that exhibit TF-independent Factor X proteolytic activity.

**Pharmacokinetic Variants**

Factor VIIa-related polypeptides encompass those for which one or more pharmacokinetic properties has been altered relative to wild type Factor VIIa.

Pharmacokinetic properties may be calculated using, e.g., WinNonlin Professional Version 3.1 (Pharsight Inc., Mountain View, CA, USA). Calculations are performed using mean concentration values at each time point, if more than one value was present.

The following pharmacokinetic parameters may be calculated: AUC, $AUC_{\%Extrap}$, $C_{max}$, $t_{max}$, $\lambda_z$, $t_{1/2}$, CL, and $V_z$ using the following formulas:

AUC  Area under the plasma concentration-time curve from time 0 to infinity. Calculated using the linear/log trapezoidal rule with extrapolation to infinity. The linear trapezoidal rule is used from time 0 to $t_{max}$:

$$AUC(0 - t_{max}) = \left( \sum_{i=1}^{n-1} \frac{C(i) + C(i+1)}{2} \cdot (t(i+1) - t(i)) \right)$$

The log trapezoidal rule is used from time $t_{max}$ to the last time point t:

$$AUC(t_{max} - t) = \left( \sum_{i=1}^{n-1} \frac{C(i) - C(i+1)}{\ln\left(\frac{C(i)}{C(i+1)}\right)} \cdot (t(i+1) - t(i)) \right)$$

Extrapolation to infinity is performed using:

$$AUC(t - \infty) = \frac{C(t)}{\lambda_z}$$

AUC$_{\%Extrap}$    Percentage of AUC that is due to extrapolation from the last concentration to infinity:

$$AUC_{\%Extrap} = \frac{AUC(t - \infty)}{AUC} \cdot 100\%$$

C$_{max}$    Maximum plasma concentration back extrapolated to time zero

CL    Total body clearance

$$CL = \frac{Dose}{AUC}$$

t$_{max}$    Time at which maximum plasma concentration is observed.

t$_{1/2}$    Half-life: $t_{1/2} = \dfrac{\ln 2}{\lambda_z}$

$\lambda_z$    Terminal rate constant. Calculated by log-linear regression of (mean) concentrations versus time

V$_z$    Volume of distribution based on the terminal phase:

$$V_z = \frac{Dose}{AUC \cdot \lambda_z}$$

Non-limiting examples of useful Factor VIIa-related polypeptides include those in which the ratio between absorption and clearance has been altered to provide increased AUC and/or $t_{1/2}$.

In some embodiments, Factor VIIa-related polypeptides are those in which the modification alters the ratio between adsorption and clearance resulting in an increase in bioavailability of at least about 25%, 50%, 75%, 100%, 125%, 150%, 200%, or 500% of the bioavailability of wild-type Factor VII.

Preparation of compound

Human purified Factor VIIa suitable for use in the present invention is preferably made by DNA recombinant technology, e.g. as described by Hagen et al., Proc.Natl.Acad.Sci. USA 83: 2412-2416, 1986 or as described in European Patent No. 200.421 (ZymoGenetics).

The Factor VII variants described herein may be produced by means of recombinant nucleic acid techniques. In general, a cloned wild-type Factor VII nucleic acid sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known (see U.S. 4,784,950, where the cloning and expression of recombinant human Factor VII is described). The bovine Factor VII sequence is described in Takeya et al., J. Biol. Chem. 263:14868-14872 (1988)). It will be understood that any conventional technique may be used to produce Factor VIIa sequence variants.

Factor VII may also be produced by the methods described by Broze and Majerus, J. Biol.Chem. 255 (4): 1242-1247, 1980 and Hedner and Kisiel, J. Clin.Invest. 71: 1836-1841, 1983. These methods yield Factor VII without detectable amounts of other blood coagulation Factors. An even further purified Factor VII preparation may be obtained by including an additional gel filtration as the final purification step.

Chemical and/or enzymatic modification of wild-type Factor VIIa or Factor VIIa sequence variants may be achieved by any means known in the art. Suitable modifications include, without limitation, chemical glycan modifications as described in EP application, EP06120000 (Novo Nordisk A/S), C-terminal modifications as described in WO2006013202, glycosyltransferase mediated modification as described in WO2006035057, dendrimer modification as described in WO2005014049, carboxypeptidase mediated terminal modification (as described, e.g., in WO2005035553-A2, WO9520039-A, and WO9838285-A), transglutaminase-mediated modification (as described, e.g., WO2005070468), autocatalytic/endopeptidase-mediated transpeptidation (as described, e.g., in WO2006013202-A2); thiol-mediated modification (as described, e.g., in WO2002077218 and PCT/EP/2006063310) and amine-mediated modification (as described, e.g., in WO02/02764.

Factor VII and Factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, Factor VII may be activated by passing it through an ionexchange chromatography column, such as Mono Q® (Pharmacia) or the like. The resulting activated Factor VII may then be formulated and administered as described below.

Pharmaceutical administration

The regimen for any patient to be treated with FVIIa as mentioned herein should be determined by those skilled in the art. The dose to be administered in therapy may depend on numerous factors, such as, e.g., the weight and the condition of the patient, and can be determined by examining different points in a matrix of treatment and correlating to clinical outcomes.

In one series of embodiments, a Factor-VIIa related polypeptide is administered subcutaneously and in an amount of about 100-100,000 units per kg body weight, such as, e.g., in an amount of about 250 - 25,000 units per kg body weight corresponding to about 5-500 μg/kg.

Factor VIIa-related polypeptide may be administered to a subject as a single dose comprising a single-dose-effective amount for treating or preventing the bleeding, or in a staged series of doses which together comprise an effective amount for treating or preventing the bleeding. An effective amount of the Factor VIIa-related polypeptide refers to the amount of Factor VIIa polypeptide which, when administered in a single dose or in the aggregate of multiple doses, or as part of any other type of defined treatment regimen, produces a measurable improvement in at least one clinical parameter associated with the bleeding. When Factor VIIa-related polypeptides with different activity are administered, an effective amount may be determined by comparing one or more biological properties (including e.g, the coagulant activity and/or one or more pharmacokinetic properties) of the Factor VIIa-related polypeptides with that of known Factor VIIa polypeptides and adjusting the amount to be administered proportionately to the predetermined effective dose for the known Factor VIIa polypeptides.

Administration of a single dose refers to administration of an entire dose of Factor VIIa-related polypeptide as a bolus over a period of less than about 5 minutes. In some embodiments, the administration occurs over a period of less than about 2.5 minutes, and, in some, over less than about 1 min. Typically, a single-dose effective amount comprises at least about 40 μg/kg human Factor VIIa-related polypeptide compared to wild-type Factor VIIa, such as, at least about 50 μg/kg, 75 μg/kg, or 90 μg/kg, or at least 150 μg/kg Factor VIIa.

In some embodiments, following administration of a single dose of Factor VIIa-related polypeptide, the subject receives no further Factor VIIa-related polypeptide for an interval of at least about 30 minutes. In some embodiments the post-administration interval is at least about 45 minutes, such as at least about 1 hour, at least about 1.5 hours, or at least about 2 hours.

In other embodiments, the subject receives Factor VIIa-related polypeptide according to the following regimen: (i) The subject receives a first amount of Factor VIIa-related polypeptide comprising at least about 40 μg/kg; (ii) after a period of at least about 30 minutes, a second amount of Factor VIIa-related polypeptide is administered, the amount comprising at least about 40 μg/kg; and (iii) after a period of at least about 30 minutes from administration of the second dose, a third amount of Factor VIIa-related polypeptide is administered, the amount comprising at least about 40 μg/kg. After a period of at least about 30 minutes following the administration of the third amount, the subject may then receive a further (fourth) amount of Factor VIIa-related polypeptide comprising at least about 40 μg/kg.

In other embodiments, the first amount of Factor VIIa-related polypeptide comprises at least about 40 μg/kg, such as at least about 80 μg/kg, such as at least about 100 μg/kg or at least about 150 μg/kg or at least 200 μg/kg or at least 300 μg/kg or at least 500 μg/kg; in other embodiments, the second amount of Factor VIIa-related polypeptide comprises at least about 75 μg/kg, such as at least about 90 μg/kg; in other embodiments, the third (and optionally fourth) amount of a Factor VIIa-related polypeptide comprises at least about 75 μg/kg, such as at least about 90 μg/kg.

In one embodiment, the first dose comprises about 200 μg/kg, the second dose about 100 μg/kg, and the third (and optionally fourth) dose about 100 μg/kg.

In other embodiments, the subject receives the second amount of a Factor VIIa-related polypeptide after a period of at least about 45 minutes from the first administration, such as at least about 1 hour, at least about 1.5 hours, at least about 2 hours, at least about 2.5 hours, or at least about 3 hours.

In one embodiment, the subject receives a first dose comprising about 200 μg/kg; after a period of about 1 hour, the subject receives a second dose comprising about 100 μg/kg, and after a period of about 3 hours from the first dose, the subject receives a third dose comprising about 100 μg/kg.

*Combination treatments:*

The present invention encompasses Disclosed herein is the combined administration of an additional agent in concert with a Factor VIIa-related polypeptide. In some embodiments, the additional agent comprises a coagulant, including, without limitation, a coagulation factor such as, e.g., Factor VIII, Factor IX, Factor V, Factor XI, or Factor XIII; or an inhibitor of the fibrinolytic system, such as, e.g., PAI-1, aprotinin, ε -aminocaproic acid or tranexamic acid.

It will be understood that, in embodiments comprising administration of combinations of Factor VIIa-related polypeptides with other agents, the dosage of the Factor VIIa-related polypeptide may on its own comprise an effective amount and additional agent(s) may further augment the therapeutic benefit to the patient. Alternatively, the combination of Factor VIIa-related polypeptide and the second agent may together comprise an effective amount. It will also be understood that effective amounts may be defined in the context of particular treatment regimens, including, e.g., timing and number of administrations, modes of administrations, formulations, etc.

Formulation of medicament

An intravenous injection is normally 5-20 ml. It is normally preferred that an injection given subcutanously is between 0.05 to 1 ml. The concentration of the FVIIa-related must therefore be relatively high in such a medicament.

The volume administered can be more than 0.01 ml, such as, e.g., 0.1-2 ml, 0.25-1.5 ml, and 0.5-1 ml.

Additives increasing the bioavailability of the FVIIa-related polypeptide are suitably organic compounds *per se*, salts thereof, emulsions or dispersions containing organic compounds *per se* or salts thereof, e.g. dispersions of polar lipids, or any combination or sequence of addition thereof. Organic compounds useful are e.g. amino acids, peptides, proteins, and polysaccharides. Peptides include dipeptides, tripeptides, oligopeptides, such as collagen and gelatine. The collagen and gelatine is preferably hydrolysed. Polysaccharides include e.g. chitosans, cyclodextrins, starch, hyaluronic acids, dextrans, cellulose, and any derivatives, combinations and/or sequence of addition thereof. The starch is preferably hydrolysed. The emulsions include oil-in-water emulsions with oil as the dispersed phase and water-in-oil dispersions with oil as the continuous phase. The oil can be of vegetable or of animal origin or synthetically produced. Suitably, the vegetable oil of the emulsions is soybean oil or safflower oil, or any combination thereof. Suitably the polar liquids are one or more phospho- lipids or glycolipids or any combination thereof. The additives increasing the bioavailability of FVIIa could be added to the formulation before drying or upon reconstitution, or can be added to a stable solution or dispersion containing FVIIa.

Before administration, one or more aqueous solutions or dispersions can be added, in any mixture or sequence, to the medicament which is a stable aqueous solution, a dispersion or in dried form.

The medicament can be in a dried form, preferably freeze-dried. Before administration, the dried product or composition can be reconstituted with an aqueous solution or a dispersion e.g. a suspension, a liposomal formulation or an emulsion. The medicament can also be a stable aqueous solution ready for administration. It can also be a dispersion, e.g. a suspension, a liposomal formulation or an emulsion.

The medicament is preferably given subcutaneously. The FVIIa activity in the formulation is preferably from about 0.1 mg/ml to about 100 mg/ml, such as, e.g., from about 0.3 mg/ml to about 25 mg/ml, from about 0.6 mg/ml to about 25 mg/ml, from about 3 mg/ml to about 25 mg/ml or from about 6 mg/ml to about 25 mg/ml.

The medicament may also comprise one or more salts in order to give an isotonic solution, e.g. NaCl, KCl, and/or it may comprise one or more other isotonicity establishing compounds, preferably in an amount of more than 1.0 mg/ml.

Calcium, or other divalent metal ions, e.g. zinc, may be used as necessary for the maintenance of the FVIIa activity. It may be added as, for example, calcium chloride, but other salts such as calcium gluconate, calcium glubionate or calcium gluceptate may also be used. The medicament preferable comprises calcium chloride in an amount of more than 0.15 mg/ml.

An amino acid is preferably used to buffer the system and it also protects the protein if the formulation is freeze-dried. A suitable buffer can be glycine, lysine, arginine, histidine or glycylglycine, preferred is histidine.

A non-ionic surfactant may also be present in the medicament. The surfactant is preferable chosen from block-copolymers, such as a poloxamer, e.g. poloxamer 188, or a polyoxyethylene sorbitan fatty acid ester, such as polyoxyethylene-(20)-sorbitan monolaurate or polyoxyethylene-(80)-sorbitan monooleate. Preferred are polyoxyethylene-(80)-sorbitan monooleate (Tween 80). Tween 80 is preferably used in a concentration of at least 0.01 mg/ml. The non-ionic surfactant, if used, should preferably be present in an amount above the critical micelle concentration (CMC). See Wan and Lee, Journal of Pharm Sci, 63, p. 136, 1974.

Mono- or disaccharides (e.g. sucrose), polysaccharides such as low molecular weight dextrins, or sugar alcohols (e.g. sorbitol, glycerol or mannitol) may be added. The medicament may also comprise antioxidants such as bisulfite, ascorbate gluthathione, acetylcystein, tocopherol, methionin, EDTA, citric acid, butyl hydroxy toluene and /or butyl hydroxy anisole. Complexing agents, such as EDTA and citric acid can also be present in small concentrations for stabilising the FVIIa molecules, if they exhibit a stronger affinity for destabilising metal ions than for calcium or other divalent metal ions, e.g. zn2+. The medicament may also contain cyclodextrins, in particular sulfoalkyl ether cyclodextrins (WO2005023308). Furthermore, preservatives such as benzyl alcohol, phenol, sorbic acid, parabens, m-cresol and chlorocresol may be added.

The adjuvants are generally present in a concentration of from 0.001 to 4% w/v. The pharmaceutical preparation may also contain protease inhibitors, e.g. aprotinin or tranexamic acid.

The pH of the preparation is preferably adjusted to a value in the interval of 2 - 9. Preparations having a pH from about 5.0 to about 7.5 are preferred, more preferred are preparations having a pH from about 5.0 to about 6.5, most preferred are preparations having a pH from about 5.5 to about 6.0.

Preferably, the FVIIa-related polypeptide is highly purified, i.e. has a specific activity of more than 40 IU/$\mu$g. In one embodiment, the medicament consists of

| | |
|---|---|
| rFVIIa-related polypeptide | 4 mg/ml (30,000 IU/ml) |
| Sodium chloride | 5.84 mg/ml |
| Polysorbate 80 | 0.1 mg/ml |
| Calcium chloride, 2H2O | 1.47 mg/ml |
| Histidine | 1.37 mg/ml |
| pH 6.0 | |

Conventional techniques for preparing pharmaceutical compositions are, for example, described in Remington: The Science and Practice of Pharmacy, 19th ed., 1995.

The medicaments may be sterilised by, for example, filtration through a bacteria-retaining filter, by incorporating sterilising agents into the medicaments, by irradiating the medicaments, or by heating the medicaments. They can also be manufactured in the form of sterile solid medicaments which can be dissolved in sterile water, or some other sterile injectable medium prior to or immediately before use.

The present invention is further illustrated by the following examples. The presented examples are meant as an illustration of the invention, not as a limitation.

**Example 1: Pharmacokinetics of haemostatic proteins after s.c. administration in minipigs**

**Animals:**

The study was performed in 20 male Gottingen minipigs from Ellegaard Gottingen Minipigs ApS, Sorø Landevej 302, DK4261, Dalmose, Denmark. The body weight was in the range of 6.9-14.5 kg. Twice daily the animals were offered water and food (200 g Atromin 9023 daily). The study was performed in a thermostated room at 21-23 °C with a 12 h cycle of light and darkness. Light was on from 06.00 to 18.00 h.

**Drugs and chemicals:**

rFVIIa, rFVIIa-5K PEG, rFVIIa-10K PEG, rFVIIa-20K PEG and rFVIIa-40K PEG was used for dosing (Table 1). The different test substances were diluted in 10 mM histidine, 100 mM NaCl and 10 mM CaCl$_2$.

**Table 1** lists the specific clot activities and doses for the compounds dosed i.v. and sc.

| | Specific activity (%) | I.v. dose (mg/kg) | S.c. dose (mg/kg) |
|---|---|---|---|
| rFVII | 100 | 0.2 | 0.5 |
| 5K PEG | 61 | 0.2 | 0.5 |
| 10K PEG | 32 | 0.2 | 0.5 |
| 20K PEG | 27 | 0.2 | 0.5 |
| 40K PEG | 12 | 0.2 | 0.5 |

**Experimental design:**

The animals were allocated to receive a single i.v. or s.c. administration of the test substances (n=2) (Table 1). The dose was 0.2 mg/kg body weight i.v. and 0.5 mg/kg s.c. corresponding to 0.1 ml/kg i.v. and 0.25 ml/kg s.c. The i.v. dose was given via a needle or a short catheter in an ear vein followed by 2 ml sterile saline. The subcutaneous injection was given on the right side of the neck, approximately 5-7 cm from the ear and 7-9 cm from the middle of the neck. The injection was given with a stopper on the needle, allowing 0.4 cm of the needle to be introduced.

**Blood and tissue sampling:**

Blood samples were taken at predose, 0.25, 0.5, 1, 2, 3, 4, 6, 8, 12, 24, 30, 48, 54, 72, 80, 96, 120, 144 and 168 h post administration. Sampling from minipigs dosed rFVIIa were ended after 48 h. The blood samples were collected by use of vacutainers (2 ml) prefilled with 0.129 M Na-citrate and kept at room temperature for max 10 minutes before centrifugation at 4000 G for 5 minutes at room temperature. Immediately after centrifugtation 0.5 ml plasma was diluted with 2 ml SPA-buffer and mixed gently but thoroughly. 500 $\mu$l diluted plasma was transferred into a Micronic tube and placed in racks. 3x 500 $\mu$l diluted plasma was transferred into three Eppendorf tubes and placed in boxes. All tubes were labeled thoroughly for identification and stored at -80°C. Following the last blood samples, the animals were sedated using Zoletil 50 Vet. and sacrificed by pentobarbital injection and discarded.

**Analytical methods:**

The concentration of rFVIIa and analogues were determined by an ELISA and the activity by a clot assay.

ELISA:

The assay was basically the same as the Factor FVII EIA Kit from DakoCytomation, but modified to have higher sensitivity and to measure rhFVIIa in pig plasma. Briefly, rhFVIIa concentrations in diluted plasma samples were determined from calibrator rows of rhFVIIa prepared in SPA-buffer supplemented with pig plasma to the same percentage as in the samples. The ELISA set-up was a direct two-site sandwich based on two anti-rhFVIIa monoclonal antibodies, one serving to capture rhFVIIa and the other to detect bound rhFVIIa. Biotinylated detector antibody was incubated with diluted plasma sample or calibrator in 96-well microtiter plates coated with the capture antibody. Following, the wells were washed and incubated with horseradish peroxidase-labelled streptavidin, washed again, and incubated with a ready-to-use TMB/peroxide peroxidase colour substrate. Finally, the colour development was stopped by addition of $H_3PO_4$ and the optical density was measured at 450 nm and at 620 nm as reference.

Clot assay:

The FVIIa activity level of rFVII conjugates was analyzed using a modified FVIIa-clot assay as according to Morrisey et al.

In short, the rFVIIa variant was measured in a one-stage clotting assay employing the extracellular domains of tissue factor (soluble TF, sTF) which possesses cofactor activity for FVIIa but fails to support activation of FVII by FXa or FIXa[i]. sTF (TF 1-209, LOd 11338-125), test samples, and calibrator were diluted in 50 mM tris, 100 mM NaCl, 1% BSA, pH 7.4 (TBS/BSA) buffer. The calibrator (rFVIIa, bulk LASA 13200-008 - 1.31 mg/ml, 32 U/ug) was diluted to range from 2 - 200 pM and the calibrator was supplemented with mouse plasma to the same percentage as in the samples. Equal volumes (25 $\mu$l) of diluted samples (or calibrator) and congenital FVII deficient plasma (Helena Biosciences) were mixed. Rabbit brain cephalin (RBC, Heptest Laboratories Inc, one vial reconstituted in 10 ml 0.9 % NaCl) (50 ul) was added to rFVIIa sample/deficient plasma and allowed to incubate for 2 min at 37°C. Coagulation was initiated by addition of 50 ul sTF/CaCl$_2$ (sTF, 29 nM; CaCl$_2$, 4.2 mM). Clot times were recorded and plotted versus the concentration of rFVIIa on log-log scales and a linear regression curve was fitted to the log-transformed data by regression analysis. Clot times for the long-acting variants were converted to rFVIIa-like concentrations (nM) based on this calibration curve.

**Analysis of results:**

Results from ELISA as well as from clot activity analysis were subjected to non-compartmental pharmacokinetic analysis using the PC based software WinNonlin (Pharsight Corporation).

**Results and discussion:**

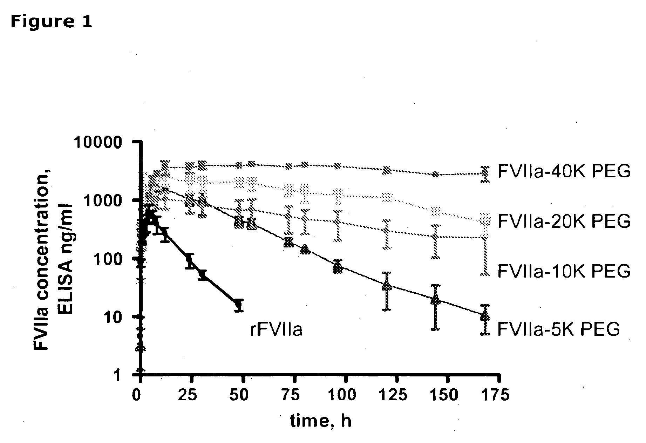

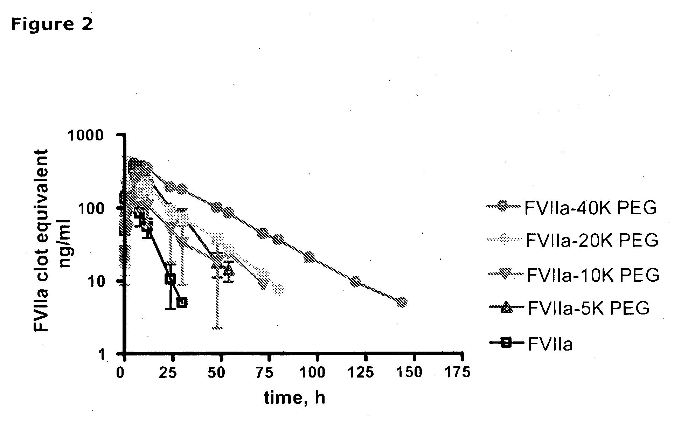

Results from the ELISA and clot assays are given in Appendix A. Figure 1 and Figure 2 illustrate the mean ($\pm$SD) of FVIIa and analogues plasma concentration determined via ELISA and clot equivalent activity versus time profile of the studied compounds, respectively.

The plasma concentration and activity profiles (Figure 1 and Figure 2) show an extended absorption phase following s.c. administration of FVIIa-5K PEG, FVIIa-10K PEG, FVIIa-20K PEg, FVIIa-40K PEG resulting in a$T_{1/2}$ of 20, 62, 33, 158 h (ELISA) and 10, 12, 14 and 21h (clot activity), respectively, compared to s.c. administration of rFVIIa resulting in a mean $T_{1/2}$ of 8h (ELISA) and 5h (clot activity) (Table 3 and Table 4).

**Example 2: Pharmacokinetics of haemostatic proteins after s.c. administration in mice**

**Animals:**

The study was performed in 82 male NMRI mice from tornbjergvej 40, Ejby, 4623 Ll. Skensved. The animals weighted approximately 30g and had free access to food and water (Altromin 1320) throughout the study period. The study was performed in a thermostated room.

**Drugs and chemicals:**

rFVIIa, FVIIa-5K PEG, FVIIa-10K PEG, FVIIa-40K PEG, FVIIa-HSA, and des-gla FVIIa, were included in the study (Table 2). FVIIa-HSA is formulated in 10 mM Glycylglycin, 150 mM NaCl, 10 mM $CaCl_2$, 0,01% Tween 80 pH 6.6, FVIIa-5K PEG and FVIIa-40K PEG are formulated in 10 mM Histidine, 150 mM NaCl, 15 mM $CaCl_2$ pH 6.0, and FVIIa-10K PEG are formulated in 10 mM Glycylglycin, 50 mM NaCl, 10 mM $CaCl_2$, pH 6.0.

The test articles were stored at -80° C until use. On the day of dosing, the test articles were thawed and stored on ice. The test articles were brought to room temperature immediately prior to dosing.

**Table 2** lists the specific clot activities and doses for the compounds dosed i.v. and sc.

| | Specific activity (%) | I.v. dose (mg/kg) | S.c. dose (mg/kg) |
|---|---|---|---|
| rFVII | 100 | 1 | 10 |
| 5K PEG | 61 | 2 | 12 |
| 10K PEG | 25 | 1 | 10 |
| 40K PEG | 13 | 1 | 14 |
| FVIIa-HSA | 40 | 1 | 7.8 |
| FVIIa-des-gla | 100 | 1 | 10 |
| | | | |
| | | | |

**Experimental design:**

The animals received a single i.v. or s.c. administration of the compounds listed in Table 1. Only a small amount of blood can be drawn from mice and thus full profiles from mice were not obtained. In the studies described below, blood sampling includes a sparse sampling regimen that allows mean profiles to be drawn based on 2-3 samples pr. time-point and 2-3 samples pr. mouse. The dose was 1-2 mg/kg body weight i.v. in the tail vein and 7.8-10 mg/kg s.c in the neck.

**Analytical methods:**

The concentration of rFVIIa was determined by an ELISA and the activity of cp-FVIIa by a clot assay.

ELISA:

The assay was basically the same as the Factor FVII EIA Kit from DakoCytoma-tion, but modified to have higher sensitivity and to measure rhFVIIa in mouse plasma. Briefly, rhFVIIa concentrations in diluted plasma samples were determined from calibrator rows of rhFVIIa prepared in SPA-buffer supplemented with mouse plasma to the same percentage as in the samples. The ELISA set-up was a direct two-site sandwich based on two anti-rhFVIIa monoclonal antibodies, one serving to capture rhFVIIa and the other to detect bound rhFVIIa. Biotinylated detector antibody was incubated with diluted plasma sample or calibrator in 96-well microtiter plates coated with the capture antibody. Following, the wells were washed and incubated with horseradish peroxidase-labelled streptavidin, washed again, and incubated with a ready-to-use TMB/peroxide peroxidase colour substrate. Finally, the colour development was stopped by addition of $H_3PO_4$ and the optical density was measured at 450 nm and at 620 nm as reference.

Clot assay:

The rFVIIa activity level of rFVIIa conjugates was analyzed using a modified FVIIa-clot assay as according to Morrisey et al

In short, the rFVIIa variant was measured in a one-stage clotting assay employing the extracellular domains of tissue factor (soluble TF, sTF) which possesses cofactor activity for FVIIa but fails to support activation of FVII by FXa or FIXa[ii]. sTF (TF 1-209, LOd 11338-125), test samples, and calibrator were diluted in 50 mM tris, 100 mM NaCl, 1% BSA, pH 7.4 (TBS/BSA) buffer. The calibrator (rFVIIa- 1.31 mg/ml, 32 U/$\mu$g) was diluted to range from 2 - 200 pM and the calibrator was supplemented with mouse plasma to the same percentage as in the samples. Equal volumes (25 $\mu$l) of diluted samples (or calibrator) and congenital FVII deficient plasma (Helena Biosciences) were mixed. Rabbit brain cephalin (RBC, Heptest Laboratories Inc, one vial reconstituted in 10 ml 0.9 % NaCl) (50 ul) was added to rFVIIa sample/deficient plasma and allowed to incubate for 2 min at 37°C. Coagulation was initiated by addition of 50 $\mu$l sTF/CaCl$_2$ (sTF, 29 nM; CaCl$_2$, 4.2 mM). Clot times were recorded and plotted versus the concentration of rFVIIa on log-log scales and a linear regression curve was fitted to the log-transformed data by regression analysis. Clot times for the long-acting variants were converted to rFVIIa-like concentrations (nM) based on this calibration curve.

**Analysis of results:**

Results from ELISA and clot activity analysis were subjected to non-compartmental pharmacokinetic analysis using the PC based software WinNonlin (Pharsight Corporation).

**Results and discussion:**

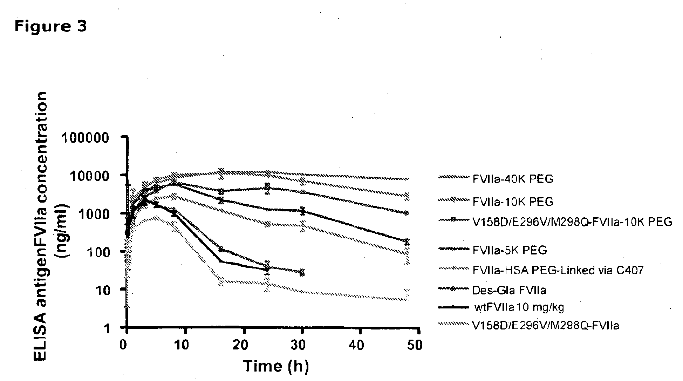

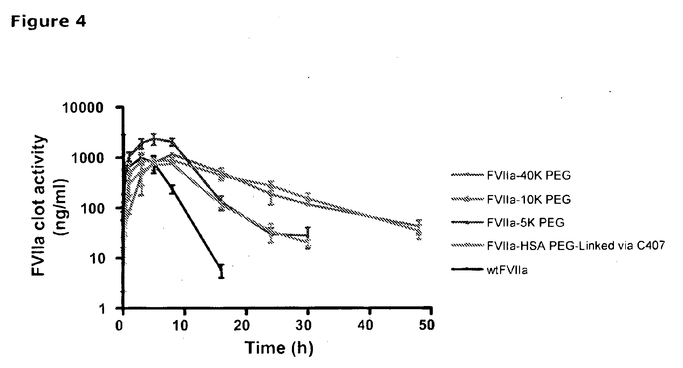

The plasma concentration and activity profiles (Figure 3 and Figure 4) show an extended absorption phase following s.c. administration of FVIIa-5K PEG, FVIIa-10K PEG, FVIIa-40K PEG and FVIIa-HSA PEG linked via C407 resulting in a mean $T_{max}$ of 8, 16, 24 and 8 h and 5, 8, 8 and 8 h and for ELISA and clot activity, respectively. The mean $T_{max}$ values of FVIIa after subcutaneous administration were 3 h for both ELISA and clot activity (Table 3 and Table 4). $C_{max}$ values and AUC were greatly reduced after s.c. administration compared to those following i.v. administration (Table 3 and Table 4). The $T_{1/2}$ of FVIIa-5K PEG, FVIIa-10K PEG, FVIIa-40K PEG and FVIIa-HSA PEG linked via C407 after s.c. administration in mice were 8.4, 14, 51, 8.2 h (ELISA) and 3.5, 8.6, 8.4, 4.2 h (clot activity) which are larger than for cpFVIIa 3.4 h (ELISA) and 1.5 h (Clot activity). The bioavailability was estimated to be 23, 78, 45 and 17 % and 12, 16, 26 and 13% for the ELISA and clot activity, respectively (Table 3 and Table 4).

Table 3 and Table 4 list the pharmacokinetic parameters obtained after a non compartmental analysis (NCA) of ELISA and clot activity data, respectively.

**Table 3** NCA data based on ELISA (mean values)

| Compound | Species | Dose (mg/kg) | | $C_{max}$ ($\mu$g/ml) | | $T_{max}$ (h) | $AUC_{0-\infty}$ (h*$\mu$g/ml) | | $AUC_{extrap.}$ (%) | | F (%) | T½ (h) | |
|---|---|---|---|---|---|---|---|---|---|---|---|---|---|
| | | i.v. | s.c. | i.v. | s.c. | s.c. | i.v. | s.c. | i.v. | s.c | s.c. | i.v. | s.c. |
| rFVIIa | | 1 | 10 | 4.5 | 2.2 | 3 | 5.7 | 16.6 | 2 | 0.3 | 29 | 1.7 | 3.4 |
| 5K PEG | | 2.1 | 12 | 15 | 5.9 | 8 | 76 | 99.6 | 2 | 2.2 | 23 | 9.1 | 8.4 |
| 10K PEG | | 1 | 10 | 6.3 | 11 | 16 | 52.9 | 411 | 3 | 14 | 78 | 15 | 14 |
| 40K PEG | Mice | 1 | 14 | 8.5 | 12 | 24 | 153 | 1034 | 16 | 57 | 48 | 28 | 51 |
| FVIIa-HSA | | 1 | 7.8 | 8.2 | 2.8 | 8 | 34.8 | 46.1 | 2 | 2.2 | 17 | 6.9 | 8.2 |
| Des-gla FVIIa | | 1 | 10 | 6.6 | 2.3 | 3 | 8.5 | 20.8 | 3 | 0 | 24 | 1.7 | 4.5 |

(continued)

| | | Dose (mg/kg) | | Cmax (μg/ml) | | Tmax (h) | AUC0-∞ (h*μg/ml) | | AUCextrap. (%) | | F (%) | T½ (h) | |
|---|---|---|---|---|---|---|---|---|---|---|---|---|---|
| rFVIIa | | 0.2 | 0.5 | 1.5 | 0.62 | 4 | 6.2 | 8.1 | 1 | 3 | 52 | 11 | 8.4 |
| 5K PEG | | 0.2 | 0.5 | 3.3 | 1.6 | 9 | 27.8 | 62.5 | 0.2 | 1 | 90 | 21 | 20 |
| 10K PEG | Minipig | 0.2 | 0.5 | 2.3 | 1.1 | 8 | 56.7 | 110 | 9 | 18 | 78 | 57 | 62 |
| 20K PEG | | 0.2 | 0.5 | 2.8 | 2.5 | 12 | 129 | 255 | 2 | 9 | 79 | 19 | 33 |
| 40K PEG | | 0.2 | 0.5 | 3.0 | 4.2 | 54 | 277 | 1350 | 19 | 54 | 195 | 59 | 158 |

**Table 1** NCA based on clot activity data (mean values)

| | | Dose (mg/kg) | | Cmax (μg/ml) | | Tmax (h) | AUC0-∞ (h*μg/ml) | | AUCextrap. (%) | | F (%) | T½ (h) | |
|---|---|---|---|---|---|---|---|---|---|---|---|---|---|
| Compound | Species | i.v. | s.c. | i.v. | s.c. | s.c. | i.v. | s.c. | i.v. | s.c | s.c. | i.v. | s.c |
| rFVIIa | | 1 | 10 | 7.5 | 1.0 | 3 | 4.4 | 6.4 | 0.6 | 0.2 | 14 | 1.5 | 1.5 |
| 5 K PEG | | 0.99 | 7.3 | 11.1 | 2.4 | 5 | 27.9 | 24 | 4 | 1 | 12 | 36.3 | 3.5 |
| 10K PEG | Mice | 0.32 | 2.1 | 2.1 | 0.88 | 8 | 6.1 | 6.3 | 0.4 | 0.2 | 16 | 3.2 | 8.6 |
| 40K PEG | | 0.13 | 1.8 | 1.0 | 1.2 | 8 | 4.8 | 17 | 1.5 | 3 | 26 | 5.1 | 8.4 |
| FVIIa-HSA | | 0.42 | 3.1 | 3.3 | 0.75 | 8 | 9.4 | 8.8 | 0.3 | 1 | 13 | 4.0 | 4.2 |
| rFVIIa | | 0.2 | 0.5 | 1.53 | 0.24 | 4 | 3.5 | 2.0 | 1 | 2 | 23 | 1.7 | 4.7 |
| 5K PEG | | 0.12 2 | 0.31 | 0.95 | 0.42 | 4 | 4.3 | 7.6 | 2 | 3 | 70 | 4.2 | 9.7 |
| 10K PEG | Mini-pig | 0.06 4 | 0.16 | 0.43 | 0.17 | 4 | 3.0 | 3.6 | 4 | 4 | 48 | 6.4 | 12 |
| 20K PEG | | 0.05 4 | 0.14 | 0.53 | 0.23 | 9 | 6.3 | 6.2 | 2 | 3 | 38 | 8.8 | 14 |
| 40K PEG | | 0.02 4 | 0.06 | 0.45 | 0.35 | 12 | 7.9 | 13 | 3 | 2 | 66 | 23 | 21 |

EMBODIMENTS DISCLOSED HEREIN

1. A method for preventing or treating a bleeding episode, the method comprising administering to a patient in need of such treatment an effective amount for such treatment of a Factor VIIa-related polypeptide, wherein the administering is via a subcutaneous or intramuscular route.

2. A method as defined in embodiment 1, wherein the Factor VIIa-related polypeptide is an amino acid sequence variant of Factor VIIa.

3. A method as defined in any of embodiments 1-2, wherein the Factor VIIa-related polypeptide comprises non-polypeptide moieties covalently or non-covalently bound to the polypeptide.

4. A method as defined in embodiment 3, wherein the non-polypeptide moiety is PEG.

5. A method as defined in any of embodiments 1-4, wherein the Factor VIIa-related polypeptide exhibits a bioavailability of at least about 125% relative to the bioavailability of wild-type Factor VIIa.

6. A method as defined in any of embodiments 1-5, wherein the Factor VIIa-related polypeptide is administered via a subcutaneous route.

7. Use of a Factor VIIa-related polypeptide for the preparation of a medicament for preventing or treating a bleeding episode, wherein said preparation is suitable for administering via a subcutaneous or intramuscular route.

8. Kit of parts comprising (i) a Factor VIIa-related polypeptide in a pharmaceutical formulation, and (ii) instructions for administering via a subcutaneous or intramuscular route for preventing or treating a bleeding episode.

**Claims**

1. A PEGylated human Factor VIIa polypeptide for the use in prevention or treatment of a bleeding episode, wherein said polypeptide is for administering via a subcutaneous or intramuscular route, and wherein the average molecular weight of the polyethylene glycol moiety is 40 KDa.

2. A PEGylated human Factor VIIa polypeptide for use according to claim 1, wherein the polypeptide is for administering via a subcutaneous route.

**Patentansprüche**

1. PEGyliertes humanes Faktor-VIIa-Polypeptid zur Verwendung bei der Prävention oder Behandlung einer Blutungsepisode, wobei das Polypeptid für die Verabreichung über eine subkutane oder intramuskuläre Route bestimmt ist und wobei das durchschnittliche Molekulargewicht der Polyethylenglykolgruppierung 40 kDa beträgt.

2. PEGyliertes humanes Faktor-VIIa-Polypeptid zur Verwendung nach Anspruch 1, wobei das Polypeptid für die Verabreichung über eine subkutane Route bestimmt ist.

**Revendications**

1. Polypeptide de Facteur VIIa humain pégylé destiné à être utilisé dans la prévention ou le traitement d'une hémorragie, ledit polypeptide étant destiné à être administré par voie sous-cutanée ou intramusculaire, et le poids moléculaire moyen du groupement polyéthylène glycol étant de 40 KDa.

2. Polypeptide de Facteur VIIa humain pégylé destiné à être utilisé selon la revendication 1, le polypeptide étant destiné à être administré par voie sous-cutanée.

**Figure 1**

**Figure 2**

**Figure 3**

**Figure 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9307890 A
- WO 9526750 A
- WO 9528954 A
- WO 0182943 A
- WO 04000366 A
- US 4784950 A
- US 5997864 A
- US 5580560 A
- DK 0200189 W
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- WO 0158935 A
- US 6806063 B
- US 20030096338 A
- WO 0393465 A
- WO 04029091 A
- WO 04083361 A
- WO 04111242 A
- WO 04108763 A
- WO 0183725 A
- WO 0222776 A
- DK 0200635 W
- WO 03027147 A
- PA 200201423
- WO 04029090 A
- PA 200101627
- JP 2001061479 B
- WO 2005014035 A
- WO 0331464 A
- US 20040043446 A
- US 20040063911 A
- US 20040142856 A
- US 20040137557 A
- US 20040132640 A
- WO 0104287 A
- US 20030165996 A
- WO 0202764 A
- US 20030211094 A
- EP 200421 A
- EP 06120000 A
- WO 2006013202 A
- WO 2006035057 A
- WO 2005014049 A
- WO 2005035553 A2
- WO 9520039 A
- WO 9838285 A
- WO 2005070468 A
- WO 2006013202 A2
- WO 2002077218 A
- EP 2006063310 W
- US 4456591 A, Thomas
- WO 2005023308 A

**Non-patent literature cited in the description**

- **PERSSON et al.** *J. Biol. Chem.,* 1997, vol. 272, 19919-19924
- **PERSSON.** *FEBS Letts.,* 1997, vol. 413, 359-363
- **LINO et al.** *Arch. Biochem. Biophys.,* 1998, vol. 352, 182-192
- **MOLLERUP et al.** *Biotechnol. Bioeng.,* 1995, vol. 48, 501-505
- **KORNFELT et al.** *Arch. Biochem. Biophys.,* 1999, vol. 363, 43-54
- **HAGEN et al.** *Proc.Natl.Acad.Sci. USA,* 1986, vol. 83, 2412-2416
- **TAKEYA et al.** *J. Biol. Chem.,* 1988, vol. 263, 14868-14872
- *J. Biol.Chem.,* 1980, vol. 255 (4), 1242-1247
- **HEDNER ; KISIEL.** *J. Clin.Invest.,* 1983, vol. 71
- **OSTERUD et al.** *Biochem.,* 1972, vol. 11, 2853
- **HEDNER et al.** *J. Clin. Invest.,* 1983, vol. 71, 1836
- **WAN ; LEE.** *Journal of Pharm Sci,* 1974, vol. 63, 136
- **REMINGTON.** *The Science and Practice of Pharmacy,* 1995